# EUROPEAN PATENT APPLICATION

(11) **EP 1 300 390 A1**
(43) Date of publication of application: **09.04.2003**
(21) Application number: 01934359.9
(22) Date of filing: 25.05.2001
(51) Int. Cl.: C07C 211/58, H05B 33/14, H05B 33/22, H01L 31/04

(54) **NOVEL TRIPHENYLAMINES AND USE THEREOF**

(30) Priority: 30.05.2000 JP 2000164764
(71) Applicant: BANDO CHEMICAL INDUSTRIES, LTD., Kobe-shi, Hyogo 652-0883 (JP); Shirota, Yasuhiko, Toyonaka-shi, Osaka 561-0827 (JP)
(72) Inventor: SHIROTA, Yasuhiko, Toyonaka-shi Osaka 561-0827 (JP); INADA, Hiroshi Bando Chemical Industries, Ltd., Kobe-shi Hyogo 652-0883 (JP); TAKAHASHI, Yoshiko Bando Chemical Industries, Ltd., Kobe-shi Hyogo 652-0883 (JP); KAMENO, Isao Bando Chemical Industries, Ltd., Kobe-shi Hyogo 652-0883 (JP)
(74) Representative: Bates, Philip Ian
(86) International application number: JP0104424
(87) International publication number: WO01092203

(57) **Abstract**

The triphenylamine having the general formula (I) 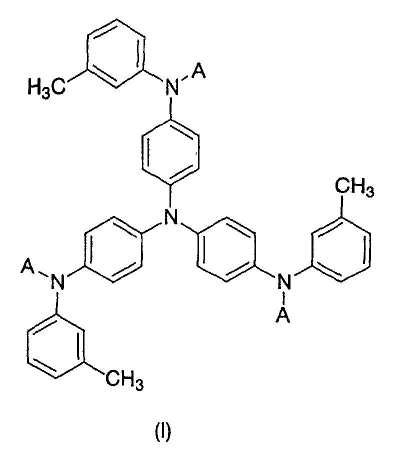 wherein A is a 1-naphthyl group or a 2-naphthyl group. It has a glass transition temperature as high as 100°C and yet it remains amorphous stably. Besides, it has good solubility in many organic solvents.

## Description

### Field of the Invention

This invention relates to novel triphenylamines useful for use as amorphous electronic materials and their use. More particularly, the invention relates to novel and useful triphenylamines that remain amorphous at normal temperatures so that they can form thin films by themselves, and in addition, that is highly heat-resistant so that they are suitable for use as organic positive hole (electric charge) transport agents in a variety of elements of electronic devices, such as organic electroluminescence elements, organic photosensitive elements, organic solar battery elements or field-effect transistors.

### Background Art

The low molecular weight organic compounds having a photoelectric function which produce electroconductivity or electric charges when being irradiated are incapable of forming thin films by themselves. Accordingly, when a thin film is to be formed with such known low molecular weight organic compounds, they are dispersed in a binder resin (that is, diluted with a binder resin), and the resulting dispersion is applied to a substrate to form a thin film. The known low molecular weight organic compounds having a photoelectric function are influenced by the binder resin which forms a matrix as well as they are diluted with the binder resin so that they cannot exhibit sufficiently the properties that they originally possess. In addition, if the known low molecular weight organic compounds having a photoelectric function form a thin film that is relatively stable at normal temperatures with the aid of a binder, they have low glass transition temperatures so that the film is poor in heat resistance and is not suitable for practical use.

Accordingly, the development of electronic materials that are amorphous at normal temperatures and are capable of forming films by themselves has been pushed on with in recent years. As a result, for example, 4,4',4"-tris(N,N-phenyl-m-tolylamino)triphenylamine (m-MTDATA; Japanese Patent Application Laid-open No.1-224353) represented by the formula (1): 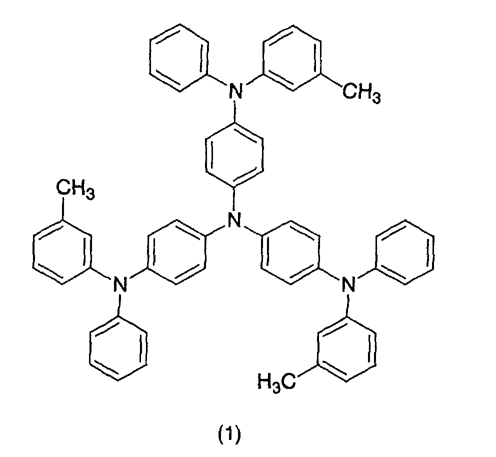 or 4,4',4"-tris(N,N-(2-naphthyl)phenylamino)triphenylamine (2-TNATA; Japanese Patent Application Laid-open No.8-291115) represented by the formula (2): 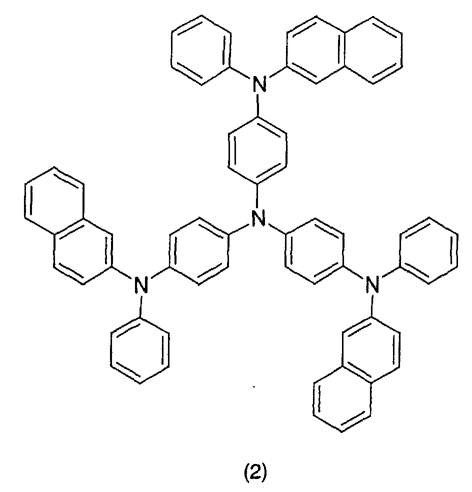 has been proposed for use as such materials. 4,4',4"-Tris(N,N-(1-naphthyl)-phenylamino)triphenylamine (1-TNATA)) has been also proposed. These triphenylamines are known to be useful as positive hole transport agents in organic electroluminescence elements ("Monthly Display", October 1998, Extra Number, pages 28-35; Japanese Patent Application Laid-open No. 5-234681).

Among a variety of electronic devices, in particular, an organic electroluminescence element is driven by a direct current at a low electric voltage with high efficiency to emit light at a high luminance, as well as it can be made thin. Accordingly, in recent years, the investigation to put the organic electroluminescence element to practical use as display devices as well as backlights or illumination devices is pushed forward.

The electroluminescence element is comprised usually of a transparent substrate such as a glass substrate having an anode made of a transparent electrode such as an ITO membrane (indium oxide-tin oxide membrane) laminated thereon, and an organic hole transport layer, an organic emitting layer and a cathode made of a metal electrode laminated on the anode in this order. The anode and the cathode are connected with an external power source. In some cases, an organic electron transport layer is laminated between the organic emitting layer and the cathode. Many other layer constructions to form organic electroluminescence elements are known, as described in, for example, Japanese Patent Application Laid-Open No. 6-1972.

In such an organic electroluminescence element, the organic positive hole transport layer adheres to the anode, and transports holes from the anode to the organic emitting layer while blocking electrons, whereas the organic electron transport layer adheres to a cathode, and transports electrons from the cathode to the organic emitting layer. Thus, when an electron infused from the cathode and a hole infused from the anode recombine in the organic emitting layer, light is emitted and radiated outside through the transparent electrode (anode) and the transparent substrate.

However, the above-mentioned 4,4',4"-tris(N,N-phenyl-m-tolylamino)triphenylamine (m-MTDATA) has a glass transition temperature of about 77°C so that it is difficult to use the compound in practical electronic devices such as organic electroluminescence elements. On the other hand, the above-mentioned 4,4',4"-tris- (N,N-(2- or 1-naphthyl)-phenylamino)triphenylamine (2- or 1-TNATA) has a glass transition temperature of about 110°C and forms a heat-resistant amorphous membrane, however, it is apt to form crystals and it is hard to say that it remains amorphous stably. Furthermore, such known triphenylamines as above-mentioned are usually only slightly soluble in many organic solvents. Accordingly, a large quantity of organic solvents are needed to purify crude products of such triphenylamines as produced, which is a problem when they are to be produced in an industrial scale.

The invention has been achieved to solve such problems of the known triphenylamines for use as amorphous electronic materials. Accordingly, it is an object the invention to provide novel triphenylamines that have a glass transition temperature of not less than 100°C and yet it remains amorphous stably, and that, in addition, has good solubility in many organic solvents.

### Disclosure of the Invention

The invention provides triphenylamines having the general formula (I): 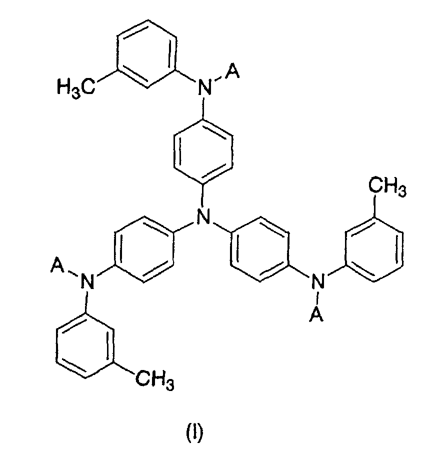 wherein A represents a 1-naphthyl group or a 2-naphthyl group.

### Brief Explanation of Drawings

Fig. 1 is an FT-IR spectrum of 4,4',4"-tris[N,N-(1-naphthyl)-m-tolylamino]triphenylamine (1-MTNATA) of the invention;
Fig. 2 is a DSC chart of 4,4',4"-tris[N,N-(1-naphthyl)-m-tolylamino]triphenylamine (1-MTNATA) of the invention;
Fig. 3 is a CV chart of 4,4',4"-tris[N, N-(1-naphthyl)-m-tolylamino]triphenylamine of the invention (1-MTNATA);
Fig. 4 is an FT-IR spectrum of 4,4',4"-tris[N,N-(2-naphthyl)-m-tolylamino]triphenylamine of the invention (2-MTNATA);
Fig. 5 is a DSC chart of 4,4',4"-tris[N,N-(2-naphthyl)-m-tolylamino]triphenylamine of the invention (2-MTNATA);
Fig. 6 is a CV chart of 4,4',4"-tris[N,N-(2-naphthyl)-m-tolylamino]triphenylamine of the invention (2-MTNATA);
Fig. 7 is a DSC chart of 4,4',4"-tris[N,N-phenyl-m-tolylamino]triphenylamine (m-MTDATA);
Fig. 8 is a DSC chart of 4,4',4"-tris[N,N-(1-naphthyl) phenylamino]triphenylamine (1-MTNATA); and
Fig. 9 is a DSC chart of 4,4',4"-tris[N,N-(2-naphthyl) phenylamino]triphenylamine (2-MTNATA).

### Best Mode for Carrying out the Invention

The triphenylamine of the invention is obtainable by reacting 4,4',4"-triiodotriphenylamine with m-tolyl-1- or 2-naphthylamine corresponding to the desired triphenylamine in a solvent in the presence of a base and a copper powder. As the base, an alkali metal hydroxide such as potassium hydroxide or sodium hydroxide is preferably used while as the solvent, a hydrocarbon such as mesitylene is preferably used, although the base and solvent used are not limited to these exemplified.

The novel triphenylamine of the invention has features that the triaminotriphenyl amine structure in the center of the molecule decreases the oxidation potential of the molecule to improve the efficiency of charge injection and charge transport; the asymmetrical structure composed of m-tolyl and naphthyl groups forming external marginal of the molecule improves formation ability of amorphous film; and the rigidity of naphthyl group makes the glass transition temperature as high as more than 100°C. Hence, the triphenylamine of the invention has improved heat resistance.

The fact that the triphenylamine of the invention has a glass transition temperature or has no clear peak in a powder X-ray diffraction measurement proves that it has no anisotropy and is amorphous.

The triphenylamine of the invention is useful for, for example, as an organic hole transport agent, and it may be used alone or as a mixture with aforesaid 4,4',4"-tris(N,N-(2-naphthyl)phenylamino)triphenylamine (2-TNATA) or 4,4',4"-tris(N,N-(1-naphthyl)phenylamino)triphenylamine (1-TNATA).

### Examples

Examples of the invention will now be described.

### Example 1

### (Synthesis of 4,4',4"-tris[N,N-(1-naphthyl)-m-tolylamino]-triphenylamine (1-MTNATA))

15.0g of m-tolyl-1-naphthylamine, 8.1g of 4,4',4"-triiodotriphenylamine, 7.2g of potassium hydroxide, 5.0g of copper powder and 30ml of mesitylene were placed in a three necked flask and the reaction was carried out at a temperature of 180 °C for 24 hours under a nitrogen atmosphere. After the reaction, ethanol was added to the resultant reaction mixture to effect reprecipitation and the precipitate was dissolved in toluene. The resulting solution was subjected to silica gel chromatography, and the reaction product was fractionated. The reaction product was then recrystalized twice from a toluene/ethanol mixed solvent to provide 6.9g of 4,4',4"-tris[N,N-(1-naphthyl)-m-tolylamino]triphenylamine as a yellow solid. The yield was 56.4%.

| Elemental analysis (%): | | | |
|---|---|---|---|
| | C | H | N |
| Calculated | 88.24 | 5.79 | 5.97 |
| Measured | 88.01 | 5.87 | 5.95 |

Mass analysis: M(m/e)=938
FT-IR (Fourier transform infrared spectroscopy, K Br pellet method):

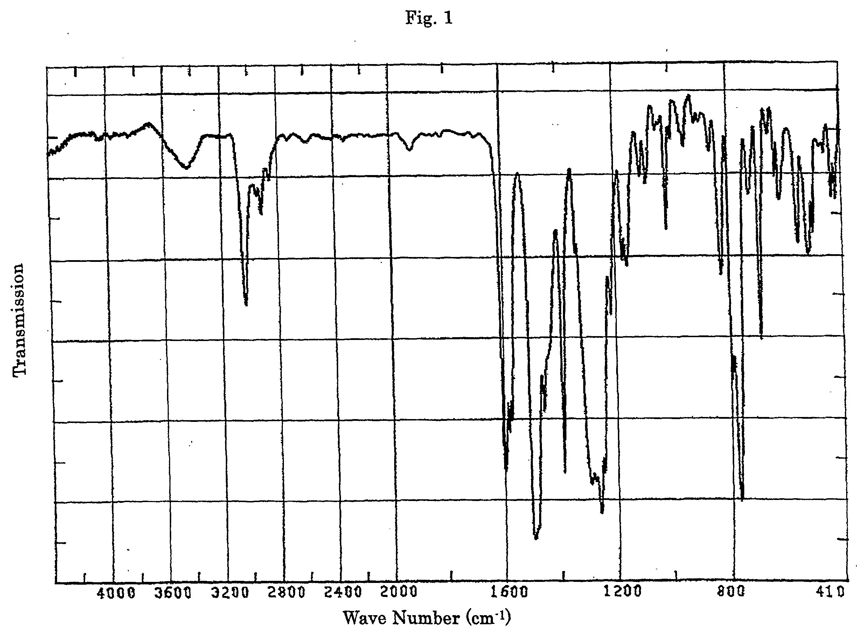

The spectrum is shown in Fig. 1.

### Differential scanning calorimetry (DSC):

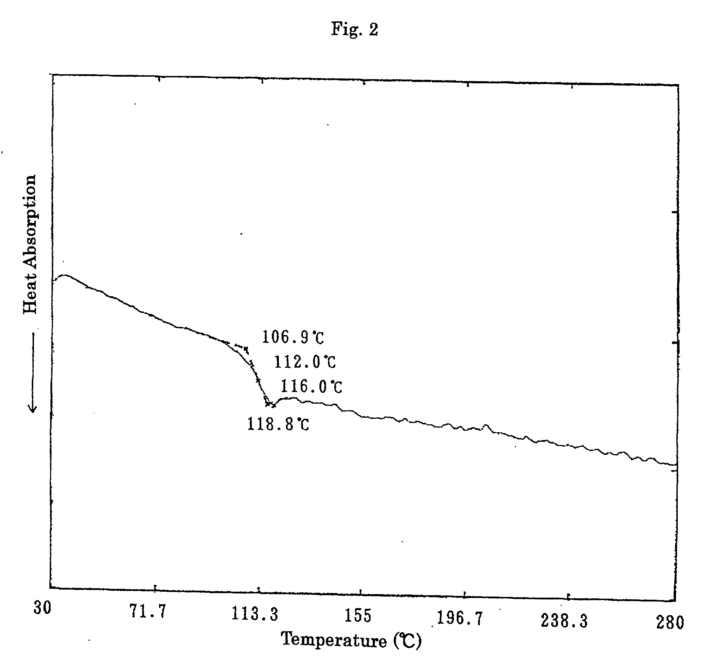

As the DSC chart is shown in Fig. 2, the compound has no peak of crystallization and remains amorphous stably. The glass transition temperature (Tg) is 106 °C and is superior in heat resistance.

### Cyclic voltammetry (CV):

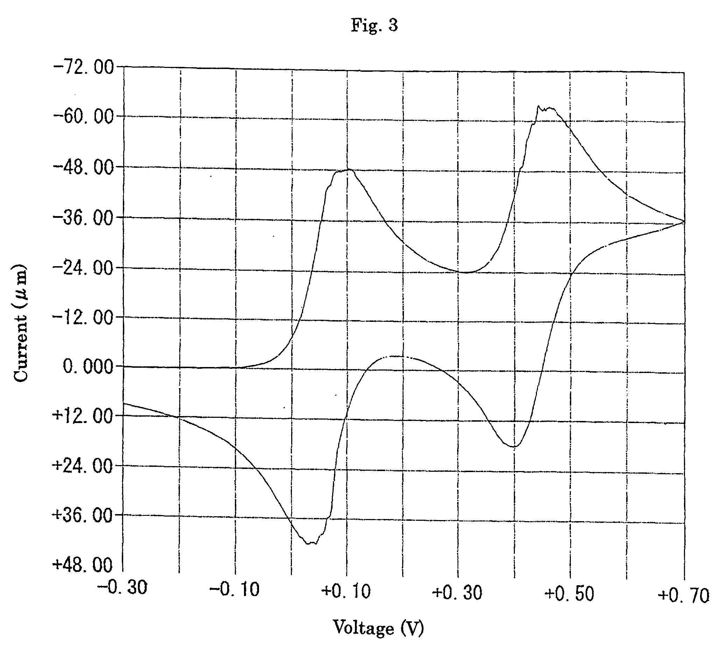

The CV chart is shown in Fig. 3. The compound has an oxidation potential 0.07V (vs. Ag/Ag⁺) to indicate that the compound is suitable for use as a hole transport agent.

### Example 2

### (Synthesis of 4,4',4"-tris[N,N-(2-naphthyl)-m-tolylamino]-triphenylamine (2-MTNATA))

15.2g of m-tolyl-2-naphthylamine, 8.3g of 4,4',4"-triiodotriphenylamine, 7.5g of potassium hydroxide, 3.0g of copper powder and 30ml of mesitylene were placed in a three necked flask and the reaction was carried out at a temperature of 180 °C for 24 hours under a nitrogen atmosphere. After the reaction, ethanol was added to the resultant reaction mixture to effect reprecipitation and the precipitate was dissolved in toluene. The resulting solution was subjected to silica gel chromatography, and the reaction product was fractionated. The reaction product was then recrystalized twice from a toluene/ethanol mixed solvent to provide 9.6g of 4,4',4"-tris[N,N-(2-naphthyl)-m-tolylamino]triphenylamine as a yellow solid. The yield was 76.9%.

| Elemental analysis (%): | | | |
|---|---|---|---|
| | C | H | N |
| Calculated | 88.24 | 5.79 | 5.97 |
| Measured | 88.07 | 5.70 | 6.03 |

Mass analysis: M(m/e)=938
FT-IR (Fourier transform infrared spectroscopy, K Br pellet method):

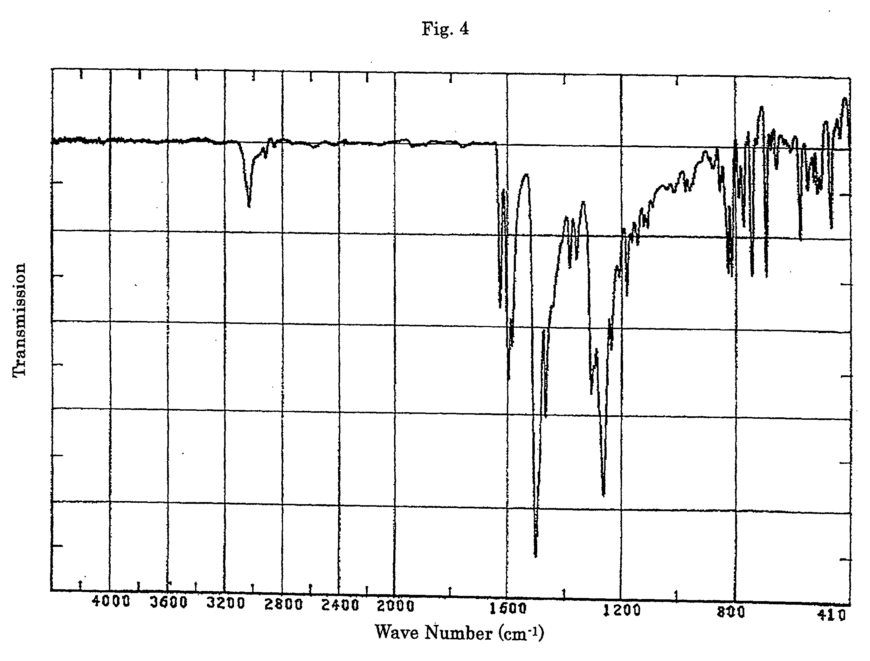

The spectrum is shown in Fig. 4.

### Differential scanning calorimetry (DSC):

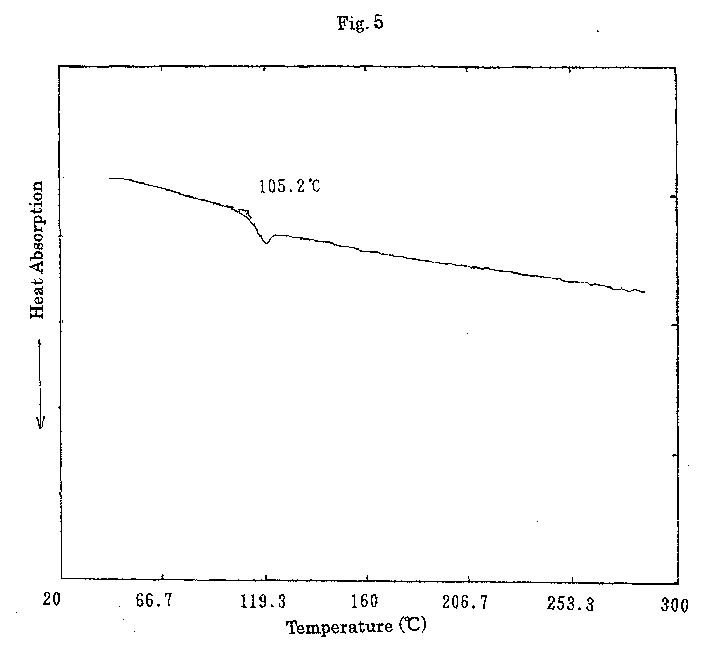

As the DSC chart is shown in Fig. 5, the compound has no peak of crystallization and remains amorphous stably.

The glass transition temperature (Tg) is 105°C and is superior in heat resistance.

### Cyclic voltammetry (CV):

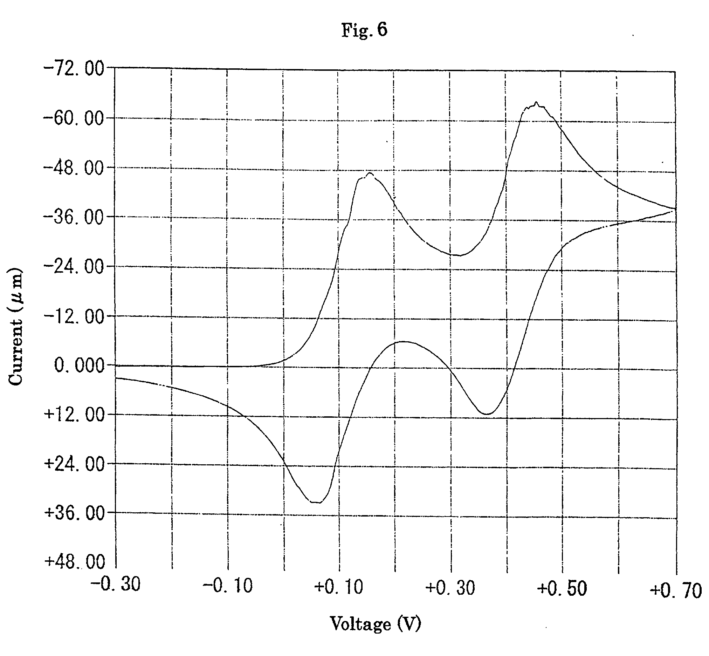

The CV chart is shown in Fig. 6. The compound has an oxidation potential 0.11V (vs. Ag/Ag⁺) to indicate that the compound is suitable for use as a hole transport agent.

### Example 3

A layer of 1-MTNATA (having a thickness of 50nm) as an organic hole transport layer, a layer of 4,4'-bis(N,N-*α*-naphthyl-m-tolylamino)diphenylamine (α-NPD) (having a thickness of 10nm) and a layer of tris(8-quinolinol) aluminum (Alq₃) (having a thickness of 50nm) as an emitting layer (having a thickness of 50nm) were laminated in this order on a transparent ITO electrode (anode) by a vacuum deposition process. Magnesium-silver alloy was co-deposited as a cathode to provide an organic electroluminescence element. A voltage was applied between the electrodes to measure the luminance of the element. The results are shown in Table 1.

### Example 4

In place of a layer of 1-MTNATA, a layer of 2-MTNATA (having a thickness of 50nm) was used as an organic hole transport layer, and otherwise in the same manner as in Example 3, an organic electroluminescence element was prepared. A voltage was applied between the electrodes to measure the luminance of the element. The results are shown in Table 1

### Comparative Example 1

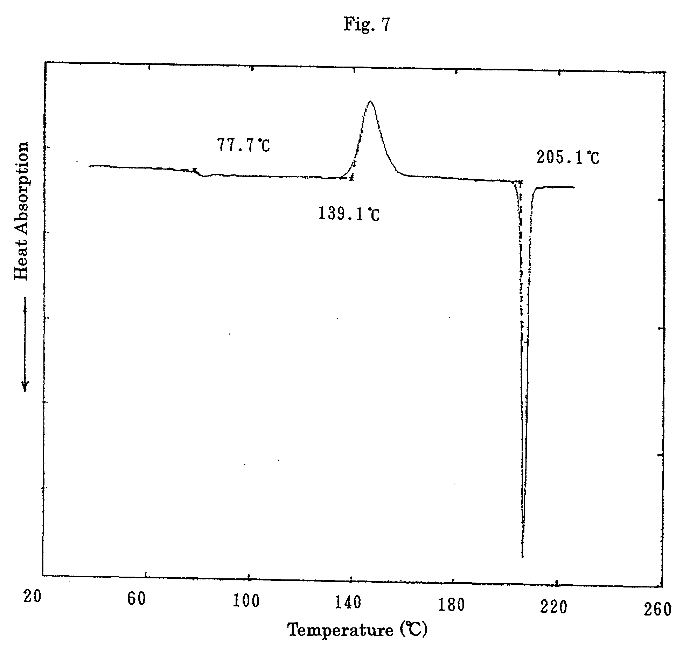

As the DSC chart of 4,4',4"-tris[N,N-phenyl-m-tolylamino]triphenylamine (m-MTDATA) is shown in Fig. 7, the compound has a glass transition temperature of about 77°C.

### Comparative Example 2

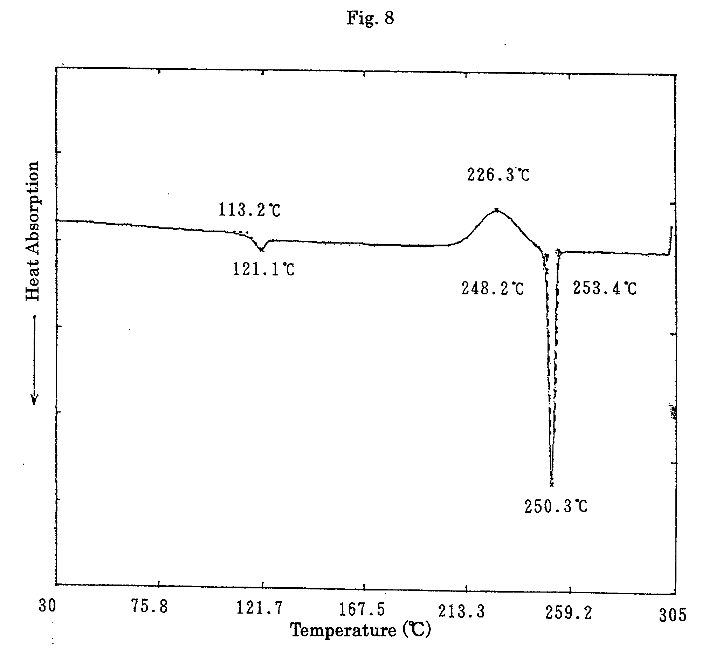

As the DSC chart of [4,4',4"-tris[N,N-(1-naphthyl)-phenylamino]triphenylamine (1-TNATA) is shown in Fig. 8, the compound has a glass transition temperature of about 113°C, however, it has a large peak of crystallization so that it is apt to form crystals.

### Comparative Example 3

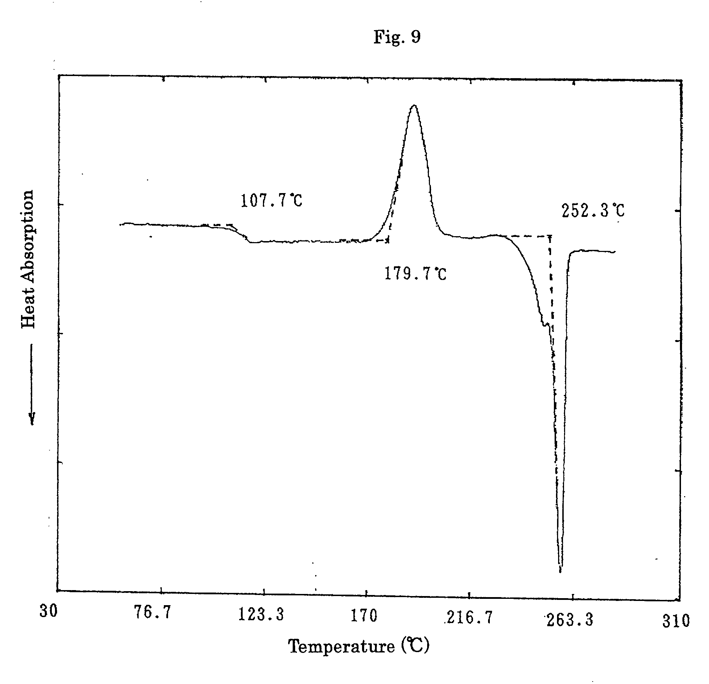

As the DSC chart of [4,4',4"-tris[N,N-(2-naphthyl)-phenylamino]triphenylamine (2-TNATA) is shown in Fig. 9, the compound has a glass transition temperature of about 113°C, however, it has a large peak of crystallization so that it is apt to form crystals.

### Comparative Example 4

In place of the organic hole transport layer of 1-MTNATA, a layer of 2-TNATA (having a thickness of 50nm) was used, and otherwise in the same manner as in Example 3, an organic electroluminescence element was prepared. A voltage was applied between the electrodes to measure the luminance of the element. The results are shown in Table 1

### Comparative Example 5

A layer of α -NPD (having a thickness of 60nm) as an organic hole transport layer and a layer of Alq₃ (having a thickness of 60nm) as an emitting layer (having a thickness of 50nm) were laminated in this order on a transparent ITO electrode (anode) by a vacuum deposition process. Magnesium-silver alloy was co-deposited as a cathode to provide an organic electroluminescence element. A voltage was applied between the electrodes to measure the luminance of the element. The results are shown in Table 1.

**Table 1**

| Voltage (V) | Luminance (cd/m²) | | | |
|---|---|---|---|---|
| | Example 3 | Example 4 | Comparative Example 4 | Comparative Example 5 |
| 2 | 0 | 0 | 0 | 0 |
| 3 | 0.01 | 0.07 | 0.03 | 0 |
| 4 | 1.3 | 1.4 | 1.3 | 0.2 |
| 5 | 9.5 | 11 | 10.1 | 2.3 |
| 6 | 42 | 49 | 44 | 10.2 |
| 7 | 136 | 153 | 148 | 25 |
| 8 | 352 | 380 | 335 | 86 |
| 9 | 810 | 806 | 798 | 175 |
| 10 | 2250 | 2320 | 2270 | 280 |
| 11 | 8600 | 8860 | 8650 | 482 |
| 12 | 16800 | 17250 | 17110 | 1230 |

As described above, the novel triphenylamines of the invention, namely, 4,4',4"-tris[N,N-(1-naphthyl)-m-tolylamino]triphenylamine (1-MTNATA) and 4,4',4"-tris[N,N-(2-naphthyl)-m-tolylamino]triphenylamine (2-MTNATA) function as organic hole transport agents effectively. Thus, for example, an organic electroluminescence element having an organic hole transport layer comprised of such a triphenylamine is driven at a lower voltage compared with the organic electroluminescence element having an organic hole layer comprised of α -NPD as prepared in Comparative Example 5.

Furthermore, an organic electroluminescence element having an organic hole transport layer comprised of the triphenylamine of the invention has the same performance as the organic electroluminescence element having an organic hole layer comprised of 2-TNATA as prepared in Comparative Example 4, and besides, the triphenylamine of the invention remains amorphous more stably than 2-TNATA, and it is useful to improve reliability of electronic devices.

### Industrial Applicability of the Invention

The novel triphenylamines of the invention, that is, 4,4',4"-tris[N,N-(1-naphthyl)-m-tolylamino]triphenylamine (1-MTNATA) or 4,4',4"-tris[N,N-(2-naphthyl)-m-tolylamino]triphenylamine (2-MTNATA) remains amorphous or glass state more stably at normal temperatures than the known triphenyl- amines. Besides, they have glass transition temperatures as high as more than 100°C.

Therefore, the triphenylamines of the invention can be formed to thin films by themselves easily by making use of their stable amorphous state. For example, they can be formed to thin films easily by a casting process or a vacuum deposition process, or they can be made to films having a large area. Accordingly, the triphenylamines of the invention are useful as amorphous electronic materials and suitable for use as organic hole (charge) transport agents in a variety of electronic devices such as, for example, organic electroluminescence elements, organic photosensitive elements, organic solar battery elements or field-effect transistors.

## Claims

1. A triphenylamine having the general formula (I) 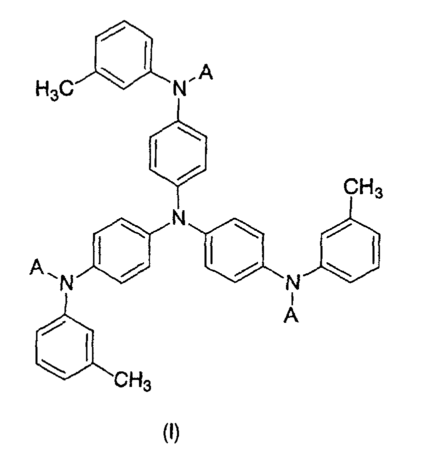 wherein A is a 1-naphthyl group or a 2-naphthyl group.

2. An organic hole transport agent comprising the triphenylamine according to claim 1.

3. An organic electroluminescence element comprising an organic hole transport layer comprising the organic hole transport agent according to claim 2.
